# EUROPEAN PATENT APPLICATION

(11) **EP 4 831 282 A1**
(43) Date of publication of application: **28.10.2026**
(21) Application number: 23962338.2
(22) Date of filing: 20.12.2023
(51) Int. Cl.: C12N 5/0786, C07D 487/22

(54) **PORPHYRIN-BASED METAL-ORGANIC FRAMEWORK-CONTAINING COMPOSITION FOR INDUCING MACROPHAGE POLARIZATION AND USE THEREOF**

(30) Priority: 19.12.2023 KR 20230186193
(71) Applicant: Mediark Inc., Seowon-gu Cheongju-si, Chungcheongbuk-do 28644 (KR)
(72) Inventor: KIM, Se Na, Sejong 30141 (KR); CHUN, Nea Young, Sejong 30128 (KR); OH, Yu Jin, Sejong 30128 (KR)
(74) Representative: Moosedog Oy
(86) International application number: PCT/KR2023/021173
(87) International publication number: WO 2025/135232

(57) **Abstract**

The present disclosure relates to a composition for inducing macrophage polarization, including a porphyrin-based metal-organic framework (MOF), and uses thereof. It has been confirmed that the composition of the present disclosure can effectively induce polarization into M2 macrophages, and therefore, the composition can be applied to a method of treating diseases associated with a mechanism of inhibiting polarization into M1 macrophages or a mechanism of inducing polarization into M2 macrophages.

## Description

### Technical Field

The present disclosure relates to a composition for inducing macrophage polarization, including a porphyrin-based metal-organic framework (MOF), and uses thereof.

### Background Art

The innate immune response is the first line of defense that protects the body from invading foreign pathogens, and is also referred to as non-specific immunity. The innate immune response involves neutrophils, monocytes, macrophages, and the like, and occurs rapidly upon pathogen invasion or infection, regardless of the type of pathogen or prior exposure to infection.

In particular, macrophages are immune cells distributed throughout all tissues of the body and are involved in the removal of invading pathogens, the elimination of virus-infected host cells and cancer cells, and the induction of inflammatory responses. Macrophages can be classified into tissue-resident macrophages differentiated from the yolk sac or fetal liver during development, and monocyte-derived macrophages differentiated from monocytes in the blood (differentiated from bone marrow cells) by inflammatory responses or pathogen invasion.

Tissue cell-derived macrophages and monocyte-derived macrophages can differentiate into M1 and M2 macrophages that function in different immune responses depending on cytokines. Cytokines are proteins involved in intercellular signaling, secreted by sequential immune responses from cells that recognize pathogens or infection, and promote the differentiation and proliferation of immune cells such as helper T cells (Th cells), B cells, and macrophages. M1 macrophages are induced by Th1 cytokines such as IFN-γ and TNF-α, and function in inducing Th1 responses, triggering inflammatory responses, inhibiting cancer growth, etc. M2 macrophages are induced by Th2 cytokines such as IL-4 and IL-10, and function in inducing Th2 responses, suppressing inflammatory responses, repairing damaged tissue, etc. As such, bone marrow-derived macrophages and monocyte-derived macrophages can differentiate into M1 or M2 macrophages having different functions depending on the type of cytokines.

These macrophages require time to be activated through cytokine-induced differentiation and proliferation. Recently, research has been actively conducted on methods of shortening the time for differentiation, proliferation, and activation of macrophages without causing reactions such as hypersensitive reactions or anaphylaxis in the human body.

Against this background, the present disclosure has been completed by developing a technology for inducing polarization into M2 macrophages by using a porphyrin-based metal-organic framework.

### Disclosure of Invention

### Technical Problem

An aspect provides a composition for inducing macrophage polarization, including a porphyrin-based metal-organic framework (MOF).

Another aspect provides a method of inducing polarization of cells in a subject, including administering the composition for inducing macrophage polarization to the subject.

Another aspect provides a method of inducing macrophage polarization, including treating isolated cells with the composition for inducing macrophage polarization.

### Solution to Problem

An aspect is to provide a composition for inducing macrophage polarization, including a porphyrin-based metal-organic framework (MOF).

The term "metal-organic framework (MOF)" as used herein refers to a porous material in which metal clusters and organic linkers or organic bridging ligands are linked by coordination bonds to form a three-dimensional structure, and various MOFs may be made according to the selection of metal ions and organic ligands. The MOF is characterized by being porous with empty spaces in the structure, and the pore size, porosity, three-dimensional structure, surface area, and the like may be designed in various ways according to the types and bonding methods of metal ions and organic ligands constituting the MOF. Due to such porosity, MOFs not only have a very large surface area but also have an open pore structure, enabling the transport of large amounts of molecules, solvents, or the like as compared to other conventionally known porous materials, and when an MOF is used as a catalyst or gas storage material, there are many active sites, thus enabling efficiency to be maximized. Moreover, the MOF is not easily deformed at high temperatures and has a rigid framework, resulting in excellent chemical stability and thermal stability.

The metal-organic framework may include a porphyrin-based compound as an organic ligand, and specifically, the porphyrin-based compound may include a compound represented by Formula 1: 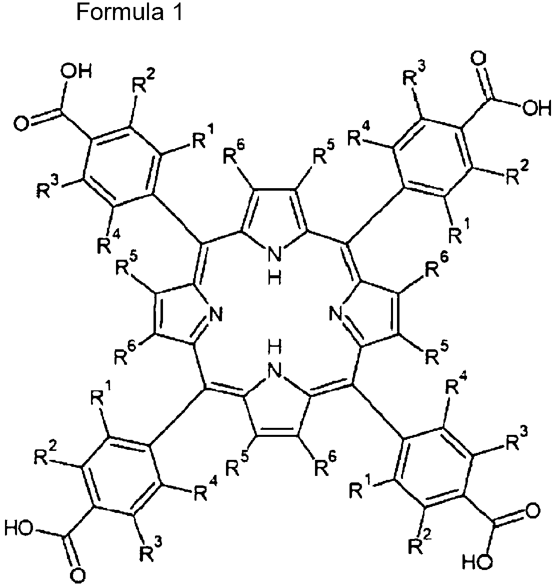 wherein R¹ to R⁴ may each be selected from hydrogen, a hydroxyl group (-OH), a halogen, a C₁-C₂₀ alkyl group, a C₂-C₂₀ alkenyl group, a C₂-C₂₀ alkynyl group, and a C₁-C₂₀ alkoxy group, R⁵ and R⁶ may each be selected from hydrogen, a halogen, a C₁-C₂₀ alkyl group, a C₂-C₂₀ alkenyl group, and a C₂-C₂₀ alkynyl group, and the C₁-C₂₀ alkoxy group may be represented by -O-R' wherein R' may be selected from a C₁-C₂₀ alkyl group, a C₂-C₂₀ alkenyl group, and a C₂-C₂₀ alkynyl group.

In an embodiment, R¹ to R⁴ in Formula 1 may each be selected from hydrogen, a hydroxyl group (-OH), a halogen, a C₁-C₂₀ alkyl group, a C₂-C₂₀ alkenyl group, a C₂-C₂₀ alkynyl group, and a C₁-C₂₀ alkoxy group. Specifically, R¹ to R⁴ may each be selected from hydrogen, a hydroxyl group (-OH), a halogen, a C₁-C₁₀ alkyl group, a C₂-C₁₀ alkenyl group, a C₂-C₁₀ alkynyl group, and a C₁-C₁₀ alkoxy group. More specifically, R¹ to R⁴ may each be selected from hydrogen, a halogen, and a C₁-C₅ alkyl group. The C₁-C₂₀ alkoxy group may be an alkyloxy group represented by -O-R' wherein R' may be selected from a C₁-C₂₀ alkyl group, a C₂-C₂₀ alkenyl group, and a C₂-C₂₀ alkynyl group.

In an embodiment, R⁵ and R⁶ in Formula 1 may each be selected from hydrogen, a halogen, a C₂-C₂₀ alkyl group, a C₂-C₂₀ alkenyl group, and a C₂-C₂₀ alkynyl group. Specifically, R⁵ and R⁶ may each be selected from hydrogen, a halogen, a C₁-C₁₀ alkyl group, a C₂-C₁₀ alkenyl group, and a C₂-C₁₀ alkynyl group. More specifically, R⁵ and R⁶ may each be selected from hydrogen and a halogen.

In an embodiment, one or more of the four carboxyl groups (-COOH) present at the terminal of the compound in Formula 1 may exist in an ionized form (-COO⁻).

In an embodiment, the porphyrin-based compound may include tetrakis(4-carboxyphenyl)porphyrin (TCPP) or an ionized form thereof.

The metal-organic framework may include one or more metals or metal ions thereof selected from the group consisting of aluminum (Al, Al²⁺_{,} Al³⁺), iron (Fe, Fe²⁺, Fe³⁺), zinc (Zn, Zn²⁺, Zn³⁺), magnesium (Mg, Mg²⁺, Mg³⁺), and zirconium (Zr, Zr²⁺, Zr³⁺_{,} Zr⁴⁺), and may be synthesized using the selected metal or metal ion. Specifically, the metal-organic framework may include the metal or metal ion and an organic ligand coordinated thereto. Specifically, the metal part of the metal-organic framework may consist of one or more metals or metal ions thereof selected from the group consisting of aluminum (Al, Al³⁺), iron (Fe, Fe³⁺), zinc (Zn, Zn²⁺, Zn³⁺), magnesium (Mg, Mg²⁺, Mg³⁺), and zirconium (Zr, Zr⁴⁺).

In an embodiment, the metal-organic framework may include zirconium (Zr, Zr²⁺, Zr³⁺_{,} Zr⁴⁺) metal or metal ions thereof. The metal-organic framework may include one or more selected from the group consisting of MOF-525 [Zr₆(OH)₄O₄(C₄₈H₂₈N₄O₈)₃], MOF-545 (PCN-222) [Zr₆O₈(C₄₈H₂₈N₄O₈)₂(H₂O)₈], PCN-221 [Zr₈O₆(C₄₈H₂₆N₄O₈)₃], PCN-223 [Zr₆O₄(OH)₄(C₄₈H₂₆N₄O₈)₃], and PCN-224 [Zr₆O₄(OH)₄(C₄₈H₂₆N₄O₈)_{1.5}].

According to an embodiment, it was confirmed that MOF-525 or MOF-545, which are porphyrin-based MOFs, can effectively induce macrophage polarization (polarization into M2 macrophages).

The term "macrophage" as used herein refers to an immune cell derived from bone marrow cells and responsible for a representative major function of innate immunity, and initially, immune cells leave the bone marrow through the bloodstream in the form of immature monocytes, and the activity of the monocytes increases during the process of recognizing pathogens or by-products derived from infected cells, leading to differentiation into mature macrophages. Macrophages have important functions in maintaining tissue homeostasis by eliminating invading foreign pathogens and inducing adaptive immunity, and play a very important role in the initial process of inflammatory responses in the human body, and specifically, depending on the method of differentiation, macrophages mature into two forms: M1 macrophages (classically activated macrophages); and M2 macrophages (alternatively activated macrophages). Among these macrophages, M2 macrophages are known to reduce inflammation and promote tissue repair.

The composition for inducing macrophage polarization refers to a composition for inducing polarization into macrophages, and specifically, may be a composition capable of regulating or inducing polarization, polar differentiation, differentiation, and/or activation into M2 macrophages (M2 phenotype).

The composition induces polarization, into M2 macrophages, of cells having the potential to be polarized or known to be polarized into M2 macrophages. Specifically, the composition may regulate or induce the polarization, polar differentiation, differentiation, and/or activation of one or more selected from the group consisting of undifferentiated macrophages, monocytes, M0 macrophages, and M1 macrophages, into M2 macrophages.

The M2 macrophages may have one or more phenotypes selected from the group consisting of CD11b+, CD45+, and Arginase 1+. Specifically, the M2 macrophages may have CD11b+, CD45+, and Arginase 1+ phenotypes.

In the composition, the metal-organic framework may further include a macrophage polarization inducer. Specifically, the metal-organic framework may be loaded with a macrophage polarization inducer.

The macrophage polarization inducer may be an active substance that induces polarization into M2 macrophages, and specifically, may be an active substance that induces polarization, polar differentiation, differentiation, and/or activation of undifferentiated macrophages, M0 macrophages, and/or M1 macrophages into M2 macrophages.

The macrophage polarization inducer may include one or more selected from the group consisting of artemisinin, interleukin (IL)-4, IL-6, IL-10, IL-13, TGF-beta, and adenosine.

In an embodiment, the metal-organic framework may be 0.001 to 500 µg/ml, specifically 0.001 to 500 µg/ml, 0.001 to 300 µg/ml, 0.001 to 200 µg/ml, 0.001 to 100 µg/ml, 0.001 to 80 µg/ml, 0.001 to 50 µg/ml, 0.001 to 30 µg/ml, 0.001 to 20 µg/ml, 0.001 to 10 µg/ml, 0.01 to 500 µg/ml, 0.01 to 300 µg/ml, 0.01 to 200 µg/ml, 0.01 to 100 µg/ml, 0.01 to 80 µg/ml, 0.01 to 50 µg/ml, 0.01 to 30 µg/ml, 0.01 to 20 µg/ml, 0.01 to 10 µg/ml, 0.1 to 500 µg/ml, 0.1 to 300 µg/ml, 0.1 to 200 µg/ml, 0.1 to 100 µg/ml, 0.1 to 80 µg/ml, 0.1 to 50 µg/ml, 0.1 to 30 µg/ml, 0.1 to 20 µg/ml, 0.1 to 10 µg/ml, 0.51 to 500 µg/ml, 0.5 to 300 µg/ml, 0.5 to 200 µg/ml, 0.5 to 100 µg/ml, 0.5 to 80 µg/ml, 0.5 to 50 µg/ml, 0.5 to 30 µg/ml, 0.5 to 20 µg/ml, 0.5 to 10 µg/ml, 1 to 500 µg/ml, 1 to 300 µg/ml, 1 to 200 µg/ml, 1 to 100 µg/ml, 1 to 80 µg/ml, 1 to 50 µg/ml, 1 to 30 µg/ml, 1 to 20 µg/ml, or 1 to 10 µg/ml, but the present disclosure is not limited thereto.

In an embodiment, the metal-organic framework may be included in an amount of, with respect to a total weight of the composition, 0.001 to 100 wt%, specifically 0.001 to 100 wt%, 0.001 to 80 wt%, 0.001 to 50 wt%, 0.001 to 30 wt%, 0.001 to 20 wt%, 0.001 to 10 wt%, 0.01 to 100 wt%, 0.01 to 80 wt%, 0.01 to 50 wt%, 0.01 to 30 wt%, 0.01 to 20 wt%, 0.01 to 10 wt%, 0.1 to 100 wt%, 0.1 to 80 wt%, 0.1 to 50 wt%, 0.1 to 30 wt%, 0.1 to 20 wt%, 0.1 to 10 wt%, 0.5 to 100 wt%, 0.5 to 80 wt%, 0.5 to 50 wt%, 0.5 to 30 wt%, 0.5 to 20 wt%, 0.5 to 10 wt%, 1 to 100 wt%, 1 to 80 wt%, 1 to 50 wt%, 1 to 30 wt%, 1 to 20 wt%, and 1 to 10 wt%, but the present disclosure is not limited thereto.

The composition may be a pharmaceutical composition.

The pharmaceutical composition may include a pharmaceutically acceptable carrier. The term "pharmaceutically acceptable carrier" may refer to a carrier or diluent that does not irritate living organisms and does not inhibit the biological activity and properties of an injected compound. "Pharmaceutically acceptable" as used herein means that the substance does not inhibit the activity of the active ingredient and does not possess toxicity beyond what the subject to whom it is administered (prescribed) can tolerate. Any type of carrier that is commonly used in the art and is pharmaceutically acceptable may be used as the carrier that may be used in the pharmaceutical composition. Non-limiting examples of the carrier include lactose, dextrose, maltodextrin, sucrose, sorbitol, mannitol, xylitol, erythritol, maltitol, glycerol, ethanol, starch, acacia gum, alginate, gelatin, calcium phosphate, calcium silicate, cellulose, methyl cellulose, microcrystalline cellulose, polyvinyl pyrrolidone, water, saline, sterile water, Ringer's solution, buffered saline, albumin injection solution, methyl hydroxybenzoate, propyl hydroxybenzoate, talc, magnesium stearate, mineral oil, or the like. These may be used alone or a mixture of two or more thereof may be used. The pharmaceutical composition may include a pharmaceutically acceptable carrier in addition to the active ingredient, and may be prepared into an oral formulation or a parenteral formulation according to a route of administration by a conventional method known in the art. The pharmaceutical composition may be formulated and used in the form of oral dosage forms such as powders, granules, tablets, capsules, suspensions, emulsions, syrups, and aerosols, or in the form of external preparations, suppositories, or sterile injectable solutions, according to conventional methods.

The pharmaceutical composition may be formulated using commonly used diluents or excipients such as fillers, extenders, binders, wetting agents, disintegrants, or surfactants, but the present disclosure is not limited thereto.

For an oral formulation, the pharmaceutical composition may be prepared into dosage forms such as powders, granules, tablets, pills, sugar-coated tablets, capsules, liquids, gels, syrups, suspensions, wafers, and the like, along with suitable carriers according to methods known in the art. Examples of suitable pharmaceutically acceptable carriers include saccharides such as lactose, glucose, sucrose, dextrose, sorbitol, mannitol, and xylitol, starches such as corn starch, potato starch, and wheat starch, celluloses such as cellulose, methyl cellulose, ethyl cellulose, sodium carboxymethyl cellulose, and hydroxypropyl methyl cellulose, polyvinyl pyrrolidone, water, methyl hydroxybenzoate, propyl hydroxybenzoate, magnesium stearate, mineral oil, malt, gelatin, talc, polyol, vegetable oil, and the like. When formulating, the pharmaceutical composition may be formulated by including diluents and/or excipients such as fillers, extenders, binders, wetting agents, disintegrants, and surfactants, as necessary.

For a parenteral formulation, the pharmaceutical composition may be formulated in the form of injections, transdermal delivery agents, nasal inhalants, and suppositories, along with suitable carriers according to methods known in the art. Examples of suitable carriers for formulation into an injection include sterile water, ethanol, polyols such as glycerol or propylene glycol, or mixtures thereof, and preferably, isotonic solutions such as Ringer's solution, phosphate buffered saline (PBS) containing triethanolamine, sterile water for injection, or 5 % dextrose may be used. For a transdermal delivery agent, the pharmaceutical composition may be formulated in the form of ointments, creams, lotions, gels, topical solutions, pastes, liniments, aerosols, and the like. For a nasal inhalant, the pharmaceutical composition may be formulated in the form of an aerosol spray using suitable propellants such as dichlorofluoromethane, trichlorofluoromethane, dichlorotetrafluoroethane, and carbon dioxide. For a suppository, witepsol, tween 61, polyethylene glycols, cacao butter, laurin butter, polyoxyethylene sorbitan fatty acid esters, polyoxyethylene stearates, sorbitan fatty acid esters, and the like may be used as bases thereof.

The pharmaceutical composition may be administered in a pharmaceutically effective amount. The term "pharmaceutically effective amount" as used herein means an amount sufficient to treat or prevent a disease at a reasonable benefit/risk ratio applicable to medical treatment or prevention, and the effective dosage level may be determined depending on factors including the severity of the disease, activity of the drug, the age, weight, health, and gender of a patient, sensitivity to the drug, administration time, administration route, and excretion rate of the composition of the present disclosure used, duration of treatment, drugs used in combination or concurrently with the composition of the present disclosure, and other factors well known in the medical field. The pharmaceutical composition of the present disclosure may be administered alone or in combination with ingredients known to exhibit therapeutic effects on known inflammation, allergic diseases, atopic dermatitis, or asthma. Taking all of the above factors into consideration, it is important to administer an amount that can achieve the maximum effect with a minimum amount without side effects.

The dosage of the pharmaceutical composition may be determined by those of ordinary skill in the art, taking into consideration the purpose of use, the severity of the disease, the age, weight, gender, and medical history of a patient, or the type of substance used as an active ingredient. For example, the pharmaceutical composition of the present disclosure may be administered in an amount of about 0.1 ng to about 1,000 mg/kg, preferably 1 ng to about 100 mg/kg, per adult, and the frequency of administration of the composition of the present disclosure is not particularly limited thereto, but the pharmaceutical composition may be administered once a day or administered several times in divided doses. The dosage or the frequency of administration does not limit the scope of the present disclosure in any aspect.

Another aspect is to provide a method of inducing polarization of cells in a subject, including administering the composition for inducing macrophage polarization to the subject. The same details as described above shall equally apply to the method.

The term " subject" as used herein may include, without limitation, mammals including dogs, cats, rats, livestock, and humans, birds, reptiles, cultured fish, and the like, and the subject may exclude humans.

The cells may be cells capable of polarization, differentiation, and/or activation into M2 macrophages, and specifically, may include one or more selected from the group consisting of undifferentiated macrophages, monocytes, M0 macrophages, and M1 macrophages.

The method may include inducing polarization, differentiation, and/or activation into M2 macrophages, and specifically, may include regulating or inducing the polarization, polar differentiation, differentiation, and/or activation of undifferentiated macrophages, monocytes, M0 macrophages, and/or M1 macrophages into M2 macrophages.

The composition may be administered in a pharmaceutically effective amount in a single dose or in multiple doses. The composition may be formulated and administered in the form of liquids, powders, aerosols, injections, infusions (Ringer's solution), capsules, pills, tablets, suppositories, or patches. The pharmaceutical composition may be administered via any general route as long as the pharmaceutical composition can reach a target tissue.

The composition is not particularly limited, but may be administered via routes such as intraperitoneal administration, intravenous administration, intramuscular administration, subcutaneous administration, intradermal administration, transdermal patch administration, oral administration, intranasal administration, intrapulmonary administration, and rectal administration, depending on the desired purpose. However, for oral administration, the composition may be administered in an unformulated form, and since the active ingredient of the composition may be denatured or degraded by gastric acid, the oral composition may be administered by coating the active ingredient, or may be administered into the oral cavity in a form formulated to be protected from decomposition in the stomach or in the form of an oral patch. In other embodiments, the composition may be administered by any device capable of delivering the active substance to target cells.

Another aspect is to provide a method of inducing macrophage polarization, including treating isolated cells with the composition for inducing macrophage polarization. The same details as described above shall equally apply to the method.

The isolated cells may be cells capable of polarization, differentiation, and/or activation into M2 macrophages, and specifically, may include one or more selected from the group consisting of undifferentiated macrophages, monocytes, M0 macrophages, and M1 macrophages.

The isolated cells may include one or more selected from the group consisting of M0 macrophages and M1 macrophages.

The method may include inducing polarization, differentiation, and/or activation into M2 macrophages, and specifically, may include regulating or inducing the polarization, polar differentiation, differentiation, and/or activation of undifferentiated macrophages, monocytes, M0 macrophages, and/or M1 macrophages into M2 macrophages.

Another aspect is to provide use of a porphyrin-based metal-organic framework (MOF) or a composition including the same for inducing macrophage polarization. The same details as described above shall equally apply to the use.

The metal-organic framework may include one or more selected from the group consisting of MOF-525 [Zr₆(OH)₄O₄(C₄₈H₂₈N₄O₈)₃], MOF-545 (PCN-222) [Zr₆O₈(C₄₈H₂₈N₄O₈)₂(H₂O)₈], PCN-221 [Zr₈O₆(C₄₈H₂₆N₄O₈)₃], PCN-223 [Zr₆O₄(OH)₄(C₄₈H₂₆N₄O₈)₃, and PCN-224 [Zr₆O₄(OH)₄(C₄₈H₂₆N₄O₈)_{1.5}].

### Advantageous Effects of Invention

It has been confirmed that the composition of the present disclosure can effectively induce polarization into M2 macrophages, and therefore, the composition can be applied to a method of treating diseases associated with a mechanism of inhibiting polarization into M1 macrophages or inducing polarization into M2 macrophages.

### Brief Description of Drawings

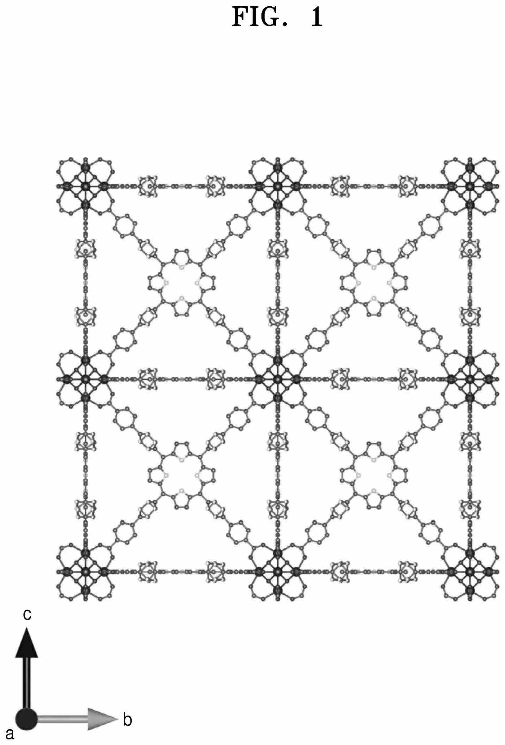

FIG. 1 is a view showing the front structure of MOF-525 particles.

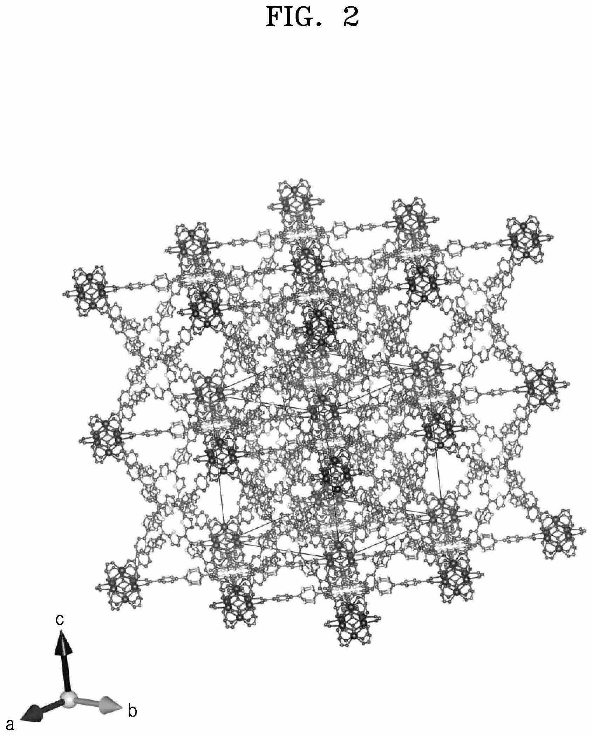

FIG. 2 is a view showing the side structure of MOF-525 particles.
FIG. 3 is an image showing the results of scanning electron microscope observation of MOF-525.
FIG. 4 is a graph showing the results of X-ray diffraction analysis of MOF-525.
FIG. 5 is a graph showing the results of measuring the adsorption-desorption isotherm and specific surface area/pore size of MOF-525.
FIG. 6 is a graph showing the results of zeta potential measurement of MOF-525.
FIG. 7 is a graph showing the results of FT-IR measurement of MOF-525.

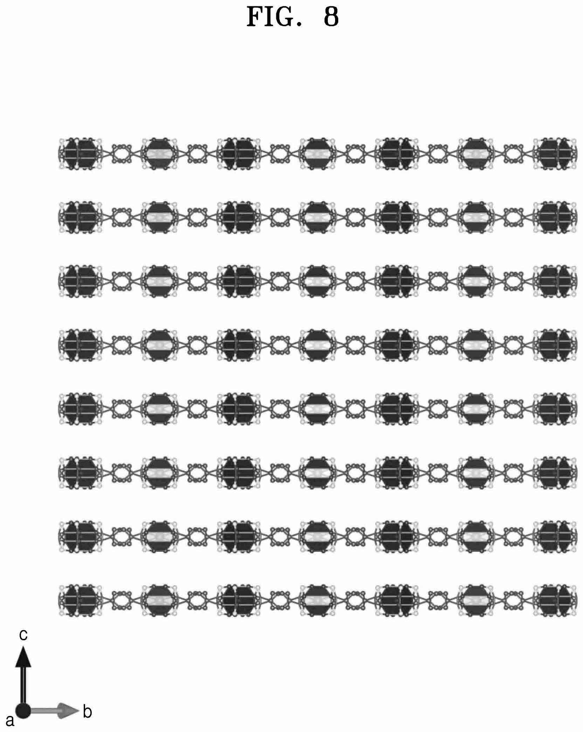

FIG. 8 is a view showing the front structure of MOF-545 particles.

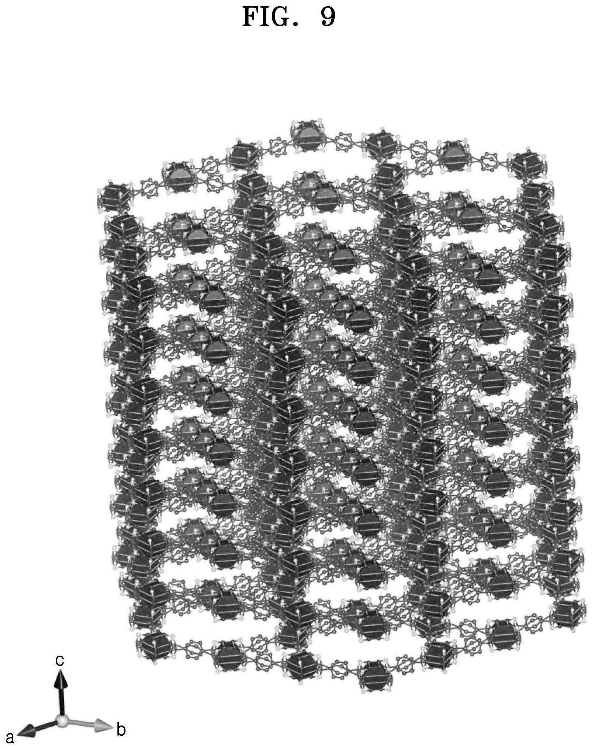

FIG. 9 is a view showing the side structure of MOF-545 particles.
FIG. 10 is an image showing the results of scanning electron microscope observation of MOF-545.
FIG. 11 is a graph showing the results of X-ray diffraction analysis of MOF-545.
FIG. 12 is a graph showing the results of measuring the adsorption-desorption isotherm and specific surface area/pore size of MOF-545.
FIG. 13 is a graph showing the results of zeta potential measurement of MOF-545.
FIG. 14 is a graph showing the results of FT-IR measurement of MOF-545.
FIG. 15 is a graph showing the results of cytotoxicity evaluation of MOF-525.
FIG. 16 is a graph showing the results of cytotoxicity evaluation of MOF-545.
FIG. 17 is a graph showing the results of evaluating the abilities of MOF-525 and MOF-545 to induce polarization into M2 macrophages.

### Mode for the Invention

Hereinafter, the present disclosure will be described in further detail with reference to the following examples. However, these examples are provided for illustrative purposes only and are not intended to limit the scope of the present disclosure.

### Example 1: Synthesis of porphyrin-based metal-organic frameworks

MOF-525 and MOF-545 were prepared/synthesized as porphyrin-based metal-organic frameworks (MOFs) by using the following method.

### 1-1: Synthesis of MOF-525

MOF-525, a porphyrin-based MOF, was synthesized as follows.

First, 130 mg of tetrakis(4-carboxyphenyl)porphyrin (TCPP) and 1.75 g of benzoic acid were added to 110 ml of N,N-dimethylformamide (DMF), followed by stirring to prepare a TCPP mixed solution. Next, 120 mg of zirconyl chloride (ZrOCl₂) was added to the TCPP mixed solution and stirred therein. Subsequently, the resulting solution was allowed to react in an oven at 100 °C for 24 hours. After the reaction, the reaction product was collected and centrifuged at 15,000 rpm for 10 minutes, and then the supernatant was removed. Next, 30 ml of DMF was added to the bottom fraction and resuspended to wash the product. Thereafter, washing was performed three times in the same manner, followed by treatment with 30 ml of ethanol three times. In the final centrifugation process, the ethanol in the supernatant was discarded, and MOF-525 particles in the bottom fraction were dried in an oven at 80 °C for 8 hours.

### 1-2: Synthesis of MOF-545

MOF-545, a porphyrin-based MOF, was synthesized as follows.

First, 32 mg of TCPP and 87 mg of zirconyl chloride (ZrOCl₂) were added to 60 ml of DMF and dissolved therein by stirring. Next, 5 ml of dichloroacetic acid was added thereto, followed by reacting at 110 °C for 18 hours. Next, washing was performed three times using DMF and ethanol as described above, followed by drying to obtain the product.

### Example 2: Confirmation of physicochemical properties of MOFs

To confirm the physicochemical properties of the MOFs prepared/synthesized in Example 1, the following experiments were performed.

First, for measurement with a scanning electron microscope (SEM), 2-3 mg of a powder sample was placed in an Eppendorf tube, and 100 µl of ethanol was added thereto, followed by bath sonication. Thereafter, 10 µl of the resultant was added dropwise onto a silicon wafer, followed by drying in an oven at 80 °C, and the silicon wafer on which the sample was dried was attached to an SEM mount using a carbon tape, and then transferred into a SEM instrument to measure an image (Manufacturer: Zeiss/Model: ULTRA PLUS).

Next, for X-ray diffraction (XRD) measurement, the powder sample was carefully placed on an XRD sample holder and compressed by pressing the sample with a glass slide with appropriate force. Thereafter, all powder scattered around the sample holder was carefully removed, the sample holder was then transferred to and set in the instrument, and measurement was performed in a range of 2-30 °C (Manufacturer: Bruker/Model: D2 Phaser).

Next, for measurement of N₂ adsorption isotherm and BET specific surface area-pore, approximately 40 mg of a completely dried sample was prepared and carefully placed into a glass sampler using a glass funnel, and then all powder adhered to the glass tube around the sample container at the bottom was removed. Thereafter, an upper filter cap was attached, and degassing was performed by vacuum and heat treatment at 120 °C for 12 hours in a pretreatment instrument. The weight of the sample after the pretreatment was measured using a microbalance, and the weight of the pre-weighed empty cell was subtracted therefrom to calculate the net weight of the sample. The glass sampler was mounted on an N2 adsorption-desorption instrument, and measurement was performed (Manufacturer: Microtrac/Model: BELSORP MINI X).

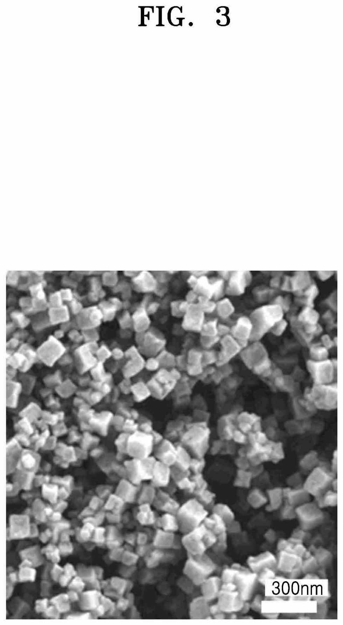

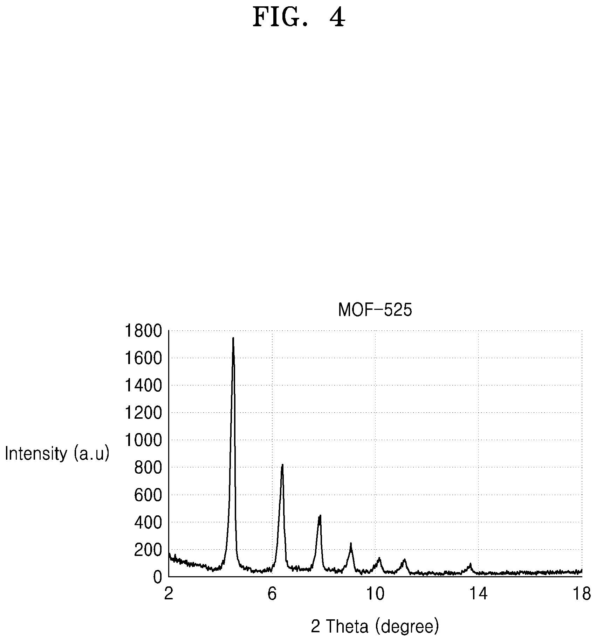

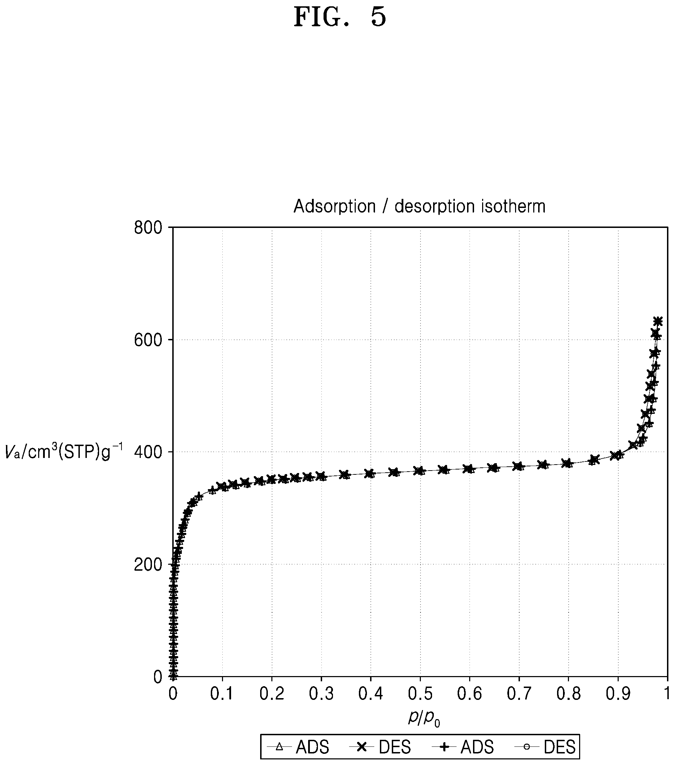

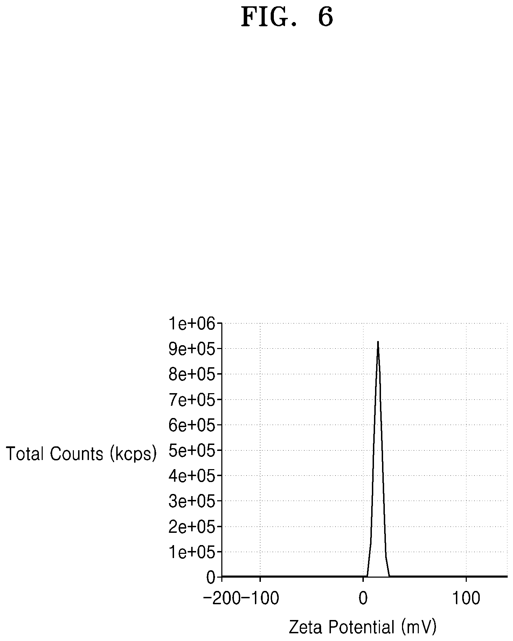

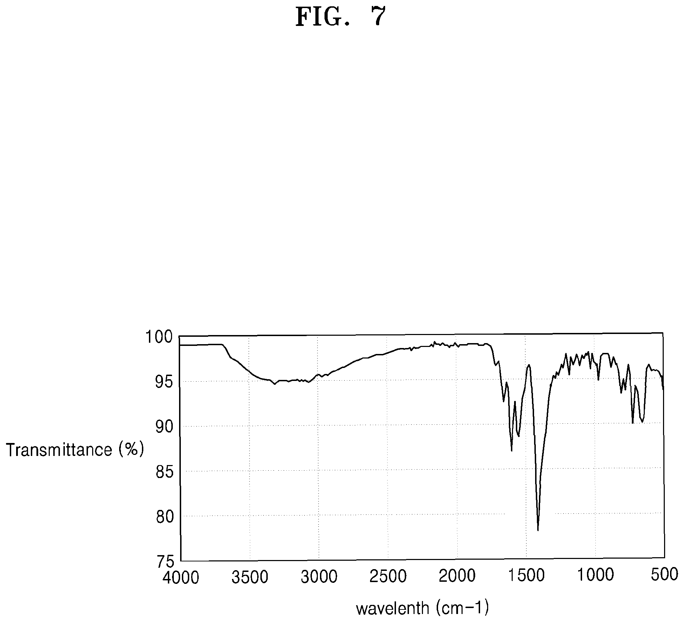

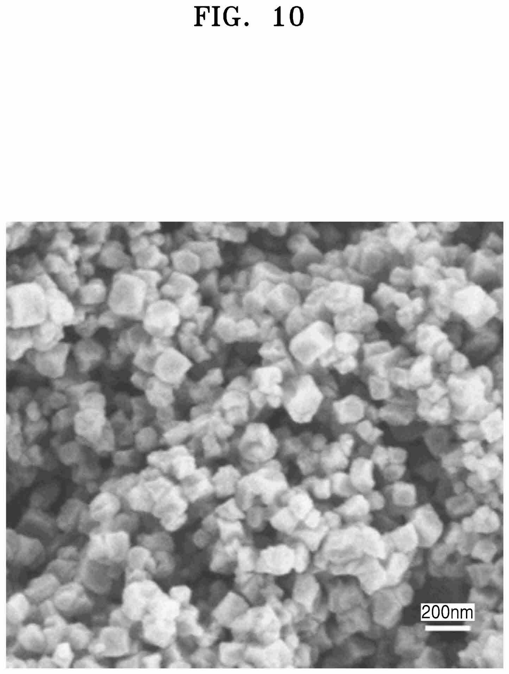

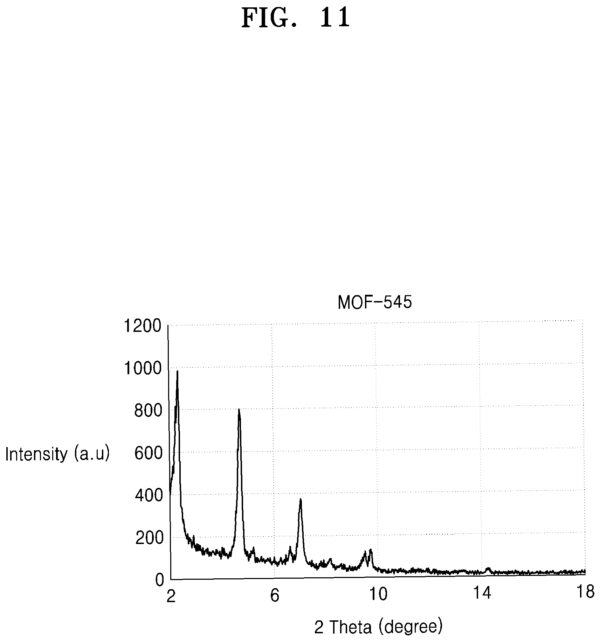

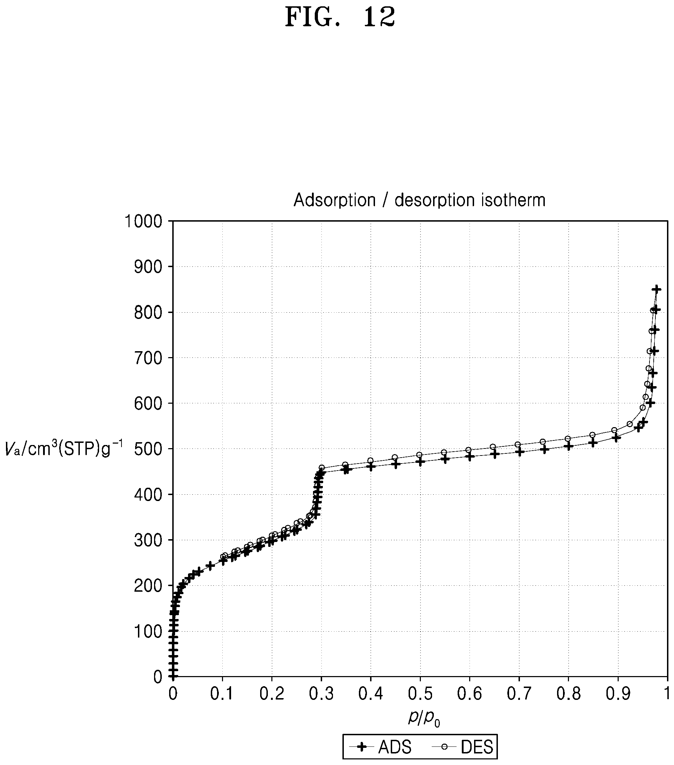

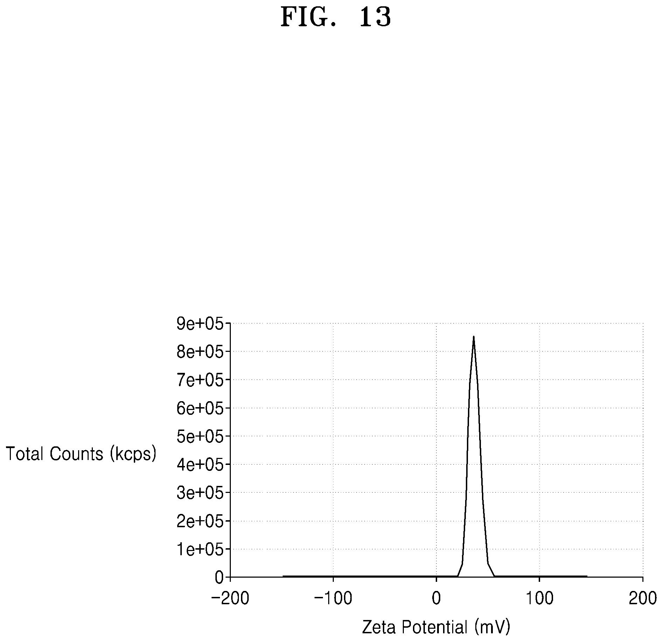

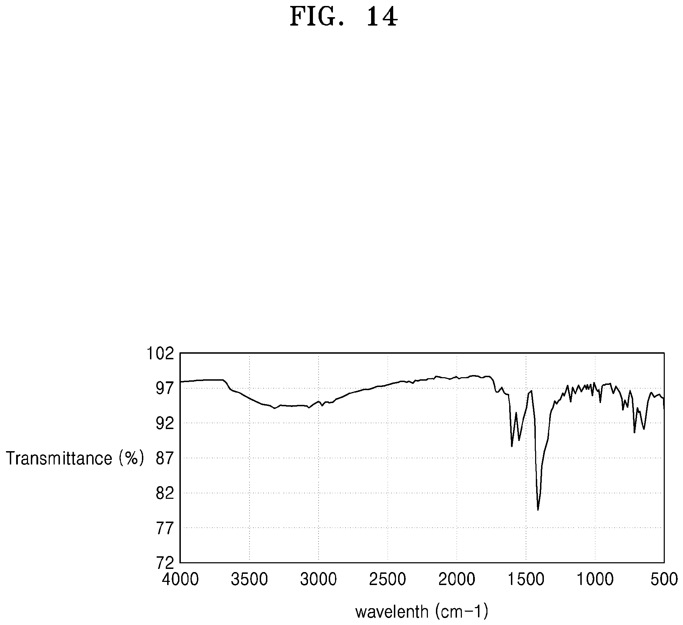

As a result of the measurement, it was confirmed that the metal-organic frameworks prepared/synthesized in Example 1 were properly synthesized (MOF-525: FIGS. 3 to 7, MOF-545: FIGS. 10 to 14).

### Example 3: Evaluation of cytotoxicity of porphyrin-based MOFs

The following experiments were performed to evaluate the cytotoxicity of the porphyrin-based MOFs.

Specifically, to evaluate the cytotoxicity of the MOFs prepared/synthesized in Example 1, mouse fibroblasts (L929) were first seeded into a 96-well plate at a density of 1 x 10⁵ cells per well, and then incubated in an incubator at a 37 °C and 5 % CO₂ for 24 hours. After 24 hours, the existing medium in each well was removed. The MOFs particles to be tested were suspended in a medium at concentrations of 50, 25, 10, or 1 µg/ml to prepare test solutions, and 100 µl of each test solution was added to each well, followed by incubation in an incubator at 37 °C and 5 % CO₂ for 24 hours, after which the medium was removed. Next, washing was performed three times with PBS to remove particles adhered to the surfaces of the plate and the cells. Next, a 10% EZ-cytox medium was added to each well, followed by reacting in an incubator at 37 °C for 1 hour, and absorbance was measured at 450 nm and 600 nm to compare the degree of cell viability with that of a control.

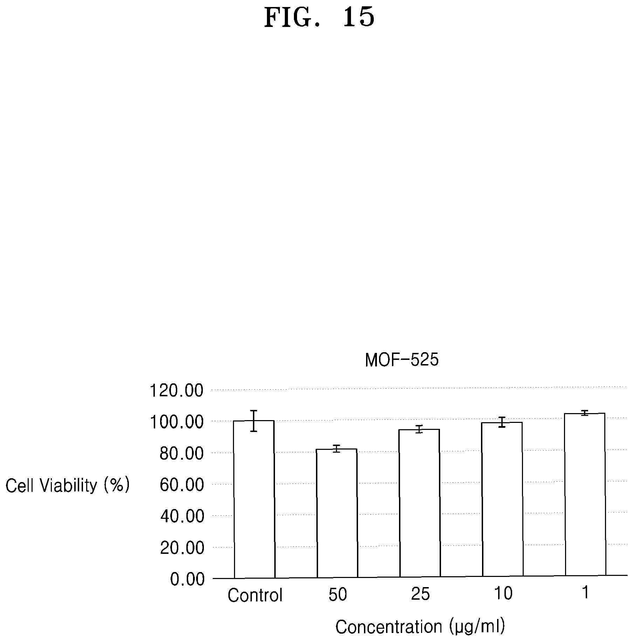

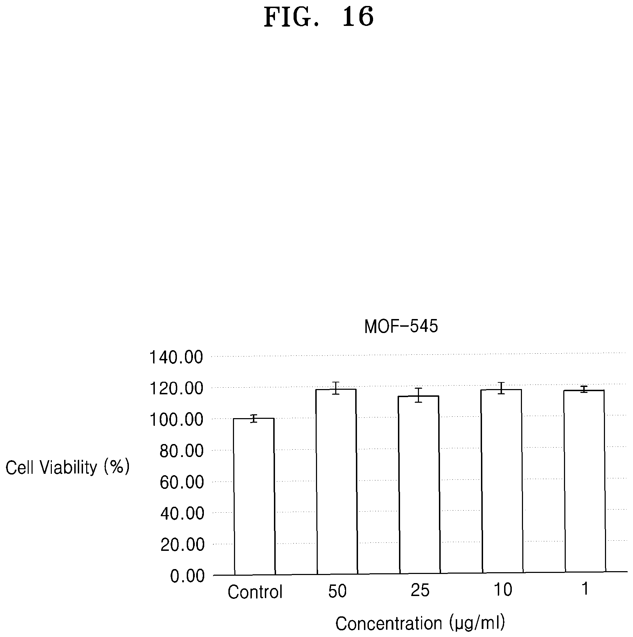

As a result of the cytotoxicity test, it was confirmed that MOF-525 and MOF-545 did not exhibit cytotoxicity at all concentrations (FIGS. 15 and 16).

### Example 4: Evaluation of macrophage polarization-inducing abilities of porphyrin-based MOFs

The following experiments were performed to evaluate the macrophage polarization-inducing abilities of the porphyrin-based MOFs.

Specifically, to evaluate the macrophage polarization-inducing abilities of MOF-525 and MOF-545, which are the MOFs prepared/synthesized in Example 1, first, bone marrow cells were collected by separating the femur and pelvis of mice, cutting the epiphysis parts, and flushing PBS through the medullary cavities to obtain undifferentiated bone marrow-derived macrophages. An ACK buffer was used to remove red blood cells from the collected bone marrow cells. Next, 10 mL of a DMEM medium and 1 mL of a L929 culture supernatant were added to a cell culture dish, and the collected bone marrow cells were cultured for 6 days, during which 10 mL of the DMEM medium was added on day 3 after the culture. The macrophages differentiated through the culture were seeded into a 96-well plate at a density of 1 x 10⁵ cells/well and cultured for 24 hours. Test samples (50 or 100 µg/mL for each sample) were added to the cultured cells, followed by incubation for 24 hours. Subsequently, quantitative analysis of unpolarized macrophages (M0) and M2-polarized macrophages was performed using a flow cytometer and fluorescent antibodies. For the quantitative analysis of the M2-polarized macrophages, the number of cells having CD11b+, CD45+, and Arginase 1+ phenotypes was calculated.

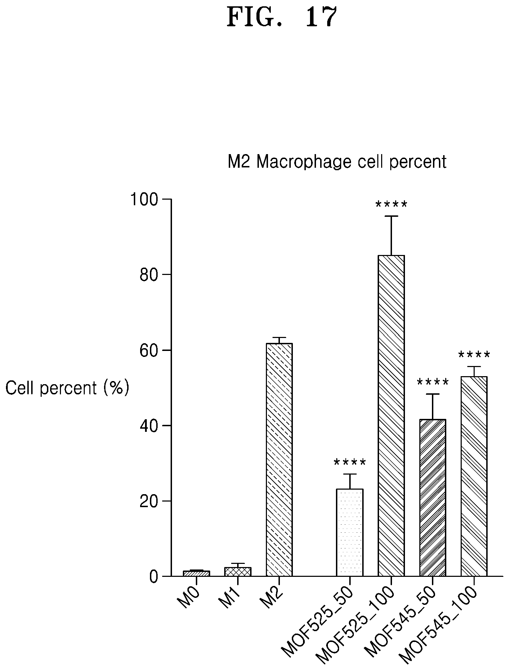

As a result of the evaluation of the macrophage polarization-inducing abilities, M2-polarized macrophages were significantly identified in the cases of MOF-525 and MOF-545 prepared/synthesized in Example 1 (FIG. 17). Therefore, it can be seen that MOF-525 and MOF-545 can effectively induce polarization into M2 macrophages, and based on this, it can be seen that porphyrin-based MOFs can effectively induce polarization into M2 macrophages.

The foregoing description of the present disclosure is provided for illustrative purposes only, and it will be understood by those of ordinary skill in the art to which the present disclosure pertains that the present disclosure may be easily modified in other particular forms without changing the technical spirit or essential characteristics of the present disclosure. Therefore, the above-described embodiments should be construed as being provided for illustrative purposes only and not for purposes of limitation.

## Claims

1. A composition for inducing macrophage polarization, comprising a porphyrin-based metal-organic framework (MOF).

2. The composition of claim 1, wherein the porphyrin-based MOF comprises a porphyrin-based compound as a ligand.

3. The composition of claim 2, wherein the porphyrin-based compound comprises a compound represented by Formula 1: 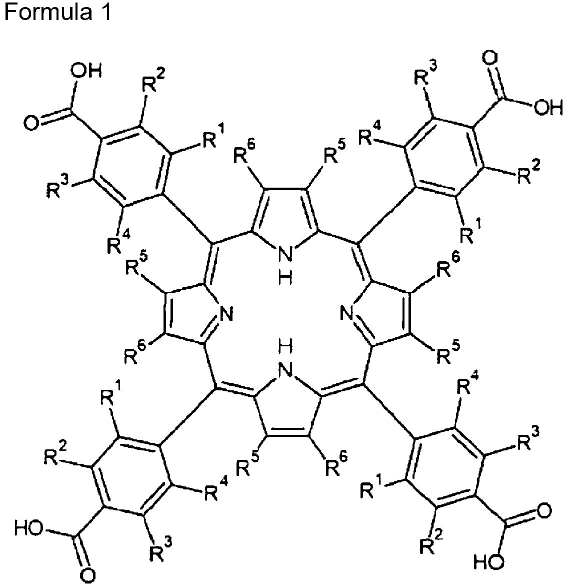 wherein R¹ to R⁴ are each selected from hydrogen, a hydroxyl group (-OH), a halogen, a C₁-C₂₀ alkyl group, a C₂-C₂₀ alkenyl group, a C₂-C₂₀ alkynyl group, and a C₁-C₂₀ alkoxy group, R⁵ and R⁶ are each selected from hydrogen, a halogen, a C₁-C₂₀ alkyl group, a C₂-C₂₀ alkenyl group, and a C₂-C₂₀ alkynyl group, and the C₁-C₂₀ alkoxy group is represented by -O-R', wherein R' is selected from a C₁-C₂₀ alkyl group, a C₂-C₂₀ alkenyl group, and a C₂-C₂₀ alkynyl group.

4. The composition of claim 1, wherein the porphyrin-based MOF comprises one or more metals or metal ions selected from the group consisting of aluminum (Al), iron (Fe), zinc (Zn), magnesium (Mg), and zirconium (Zr).

5. The composition of claim 1, wherein the porphyrin-based MOF comprises one or more selected from the group consisting of MOF-525, MOF-545 (PCN-222), PCN-221, PCN-223, and PCN-224.

6. The composition of claim 1, wherein the composition induces polarization into M2 macrophages.

7. The composition of claim 6, wherein the M2 macrophages have one or more phenotypes selected from the group consisting of CD11b+, CD45+, and Arginase 1+.

8. The composition of claim 1, wherein the porphyrin-based MOF further comprises a macrophage polarization inducer.

9. The composition of claim 8, wherein the macrophage polarization inducer comprises one or more selected from the group consisting of artemisinin, IL-4, IL-6, IL-10, IL-13, transforming growth factor-beta (TGF-beta), and adenosine.

10. A method of inducing macrophage polarization, comprising treating isolated cells with the composition of any one of claims 1 to 9.

11. The method of claim 10, wherein the isolated cells comprise one or more selected from the group consisting of undifferentiated macrophages, monocytes, M0 macrophages, and M1 macrophages.
